# EUROPEAN PATENT APPLICATION

(11) **EP 2 327 425 A1**
(43) Date of publication of application: **01.06.2011**
(21) Application number: 09177486.9
(22) Date of filing: 30.11.2009
(51) Int. Cl.: A61L 15/22, A61L 15/42

(54) **Absorbent Article**

(71) Applicant: The Procter & Gamble Company, Cincinnati, OH 45202 (US)
(72) Inventor: Carlucci, Giovanni, 66100, Chieti (IT); Gonzales, Denis Alfred, 1050, Brussels (BE)
(74) Representative: L'Huillier, Florent Charles

(57) **Abstract**

An absorbent article for feminine hygiene comprising a layered structure is provided. The article exhibits flexibility combined with shaped stability when it is worn by the user.

## Description

### FIELD OF THE INVENTION

The present invention is directed to absorbent articles for feminine hygiene and in particular to articles comprising a foam material.

### BACKGROUND OF THE INVENTION

Absorbent articles for feminine hygiene comprising foams are known in the art. The use of foams in an absorbent article is particularly advantageous on account of their liquid-absorbing capacity. Foam layers commonly used in absorbent articles typically contribute to provide form stability to the absorbent article. However, foam layers commonly used in absorbent article are exhibiting insufficient flexibility due to their relatively high thickness. This insufficient flexibility of the foam layer may be responsible for the discomfort provided to the user, since the article will not readily conform to the general shape and contours of the wearer's body. But due to the great mechanical fragility of the foam material or layer, thin layers of foam are not easily and efficiently obtainable.

Thus, there is still a need to provide an absorbent article comprising an easily processable foam layer having good absorption capacity and exhibiting flexibility combined with shape stability when the article is worn by the user.

The inventors have now found a way to solve the problem by providing an absorbent article comprising a layered structure comprising at least a foam layer and a reinforcing layer associated to the foam layer.

### SUMMARY OF THE INVENTION

The present invention addresses the above needs by providing an absorbent article for feminine hygiene comprising a layered structure, wherein the layered structure comprises a melamine foam layer and a reinforcing layer associated to the melamine foam layer.

### BRIEF DESCRIPTION OF THE DRAWINGS

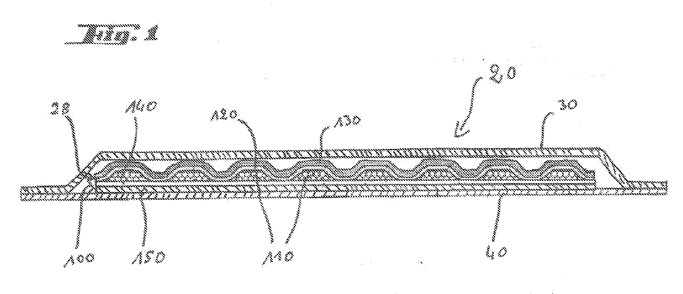

Figure 1 is a schematic cross section of a sanitary napkin comprising a layered structure according to an embodiment of the present invention.

### DETAILED DESCRIPTION OF THE INVENTION

The term "absorbent article for feminine hygiene" is used herein in a very broad sense including any article able to receive and/or absorb and/or contain and/or retain fluids and/or exudates, especially body fluids/bodily exudates such as menses. Exemplary absorbent articles for feminine hygiene in the context of the present invention are disposable absorbent articles for feminine hygiene. The term "disposable" is used herein to describe articles, which are not intended to be laundered or otherwise restored or reused as an article (i.e. they are intended to be discarded after a single use and preferably to be recycled, composted or otherwise disposed of in an environmentally compatible manner). Typical disposable absorbent articles for feminine hygiene according to the present invention are sanitary napkins, panty liners or the like. Absorbent articles suitable for use in the present invention include any type of structures, from a single absorbent layer to more complex multi layer structures. Certain absorbent articles include a fluid pervious topsheet, a backsheet, which may be fluid impervious and/or may be water vapour and/or gas pervious, and an absorbent element often called "core" comprised there between.

The term "use", as used herein with reference to absorbent articles, refers to the period of time that starts when the absorbent article is actually put in contact with the anatomy of a wearer.

By "body fluid" it is meant herein any fluid produced by human body including, but not limited to, perspiration, urine, menstrual fluids, vaginal secretions and the like.

The term "layer", as used herein with reference to absorbent articles, refers to any sheet-like element which may be comprised within the absorbent article including, but not limited to, topsheet, backsheet and absorbent element.

The term "reinforcing layer", as used herein, refers to a layer having a tear strength which is typically higher than that of the melamine foam.

The term "associated", as used herein, encompasses configurations whereby a first element is directly secured to another element by affixing the first element directly to a second element and whereby a first element is indirectly secured to a second element by affixing the first element to a third, intermediate member(s), which in turn are affixed to the second element.

The present invention relates to an absorbent article for feminine hygiene comprising a layered structure, wherein the layered structure comprises a melamine foam layer and a reinforcing layer associated to the melamine foam layer.

### Description of the melamine foam layer

Melamine foams suitable for the present invention include the one produced by the method described in US Pat. 4,666,948 or else the BASOTECT® produced by the company BASF, Germany. Melamine foams suitable for the present invention also include the one produced by the method described in US Pat. 6,800,666.

US 2005/0202232 discloses a method for making an article comprising at least one piece of sheet-form melamine having a thickness which is sufficiently small to exhibit flexibility and no flexural elasticity. An article obtained by the inventive method can be used for many applications, in particular for surface cleaning.

The inventors have surprising found that a layered structure comprising a melamine foam layer for example made according to the method disclosed in US 2005/0202232 associated to a reinforcing layer and inserted in an absorbent article will help to improve the flexibility of the absorbent article in which the layered structure is inserted. An unexpected increase of the resiliency of an absorbent article comprising the layered structure of the present invention has also been noticed.

In one embodiment, according to the disclosure of US 2005/0202232, the melamine foam layer may be formed for example by cutting a melamine foam block by peeling, using a peeling machine. Peeling method allows a cylinder of material to be cut into thin layers. For this purpose, said cylinder of material set into rotation in front of a sharpened blade is cut, the distance between the axis of rotation of said cylinder and the blade gradually decreasing during rotation of the cylinder. The thickness of the strip basically depends on the resultant between the speed of rotation of the cylinder and the speed at which the axis of rotation and the blade of the peeling machine come together.

The models of peeling machine suitable for forming a melamine foam layer from an initial melamine foam block include the ones sold by the company FECKEN-KIRFEL.

The use of a peeling machine allows the obtaining of very thin melamine foam layers. In one embodiment, the melamine foam layer has a thickness of 0.2 to 1.2 mm, or from 0.5 to 1.0 mm, or also generally less than 1.0 mm. Such a thin layer of foam will considerably help to improve the flexibility of the absorbent article.

Alternatively, a melamine foam layer of the desired thickness may be cut from a larger melamine foam block with known means.

In one embodiment, the melamine foam layer has a basis weight of 2.0 to 20.0 g/m², or from 5.0 to 12.0g/m².

### Description of the reinforcing layer

As disclosed in US 2005/0202232, a thin layer of melamine foam formed according to the method disclosed in the application may be associated with a reinforcing layer. The inventors have surprisingly found that the layered structure resulting from the association between a thin melamine foam layer and a reinforcing layer will not only help to provide flexibility to the absorbent article, i.e. capability to readily conform to the general shape and contours of the wearer, but also help to provide shape stability to the absorbent article, particularly in wet state, and under the stresses exerted during the use.

In one embodiment, the reinforcing layer can be made of a nonwoven material, for example a cellulose nonwoven, a viscose nonwoven or a perforated cotton nonwoven.

A layered structure according to this embodiment of the present invention comprising a foam layer with a reinforcing layer constituted by a nonwoven material provides an even better combination of resiliency and flexibility with mechanical resistance in a remarkably low thickness, hence with higher softness and conformability of the absorbent article comprising the structure, together with shape stability and resiliency.

In one embodiment, the reinforcing layer has a thickness of 0.2 to 4.5 mm, or equal or less than 3.0 mm, or equal or less than 2.0 mm, or also from 0.5 to 1.0 mm.

In one embodiment, the reinforcing layer has a basis weight of 10 to 400 g/m², or from 40 to 100g/m².

In one embodiment, the overall thickness of the layered structure comprising a melamine foam layer and a reinforcing layer associated to the foam layer is of 0.5 to 5.0 mm, or from 0.85 to 2.0 mm.

The melamine foam layer and the reinforcing layer may be associated to each other by using a heat activated intermediate layer, e.g a thin thermofusible intermediate layer, e.g. a thin thermofusible nonwoven. In certain cases, no heat activated intermediate layer is required, for example if the reinforcing layer itself has a capacity to bond with the foam. This is the case, for example, if the reinforcing layer is produced by printing or spraying directly on one of the faces of a foam layer, an aqueous composition adapted to dry to a film which is thus stuck directly to the foam layer. The melamine foam layer and the reinforcing layer may alternatively be associated to each other by a uniform continuous layer of adhesive, a patterned layer of adhesive, or an array of separate lines, spirals, or spots of adhesive or by pressure bonds, ultrasonic bonds, dynamic mechanical bonds, or any other suitable attachment means or combinations of these attachment means as are known in the art.

In some embodiments, absorbent articles according to the present invention comprise a topsheet, a backsheet and an absorbent core.

The topsheet may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be included of natural fibers (e.g., wood or cotton fibers), synthetic fibers (e.g., polymeric fibers such as polyester, polypropylene, or polyethylene fibers) or from a combination of natural and synthetic fibers. In one embodiment, the topsheet may be made of a hydrophobic material to isolate the wearer's skin from liquids which have passed through the topsheet. If the topsheet is made of a hydrophobic material, at least the upper surface of the topsheet is treated to be hydrophilic so that liquids will transfer through the topsheet more rapidly. This diminishes the likelihood that body exudates will flow off the topsheet rather than being drawn through the topsheet and being absorbed by the absorbent core. In one embodiment, the topsheet can be rendered hydrophilic by treating it with a surfactant. Suitable methods for treating the topsheet with a surfactant include spraying the topsheet material with the surfactant and immersing the material into the surfactant.

In one embodiment, the topsheet can be a nonwoven web that can provide reduced tendency for surface wetness; and consequently facilitate maintaining body fluids absorbed by the core away from the user's skin, after wetting. One suitable topsheet material can be a thermobonded carded web which is available as Code No. P-8 from Fiberweb North America, Inc. (Simpsonville, S.C., U.S.A.). Another suitable topsheet material is available as Code No. S-2355 from Havix Co., Japan. Yet another suitable topsheet material can be a thermobonded carded web which is available as Code No. Profleece Style 040018007 from Amoco Fabrics, Inc. (Gronau, Germany).

In another embodiment, the topsheet can include an apertured formed film. Apertured formed films can be used for the topsheet because they are pervious to body exudates and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer. Suitable formed films are described in U.S. Pat. No. 3,929,135, entitled "Absorptive Structures Having Tapered Capillaries", issued to Thompson on Dec. 30, 1975; U.S. Pat. No. 4,324,246 entitled "Disposable Absorbent Article Having A Stain Resistant Topsheet", issued to Mullane, et al. on Apr. 13, 1982; U.S. Pat. No. 4,342,314 entitled "Resilient Plastic Web Exhibiting Fiber-Like Properties", issued to Radel, et al. on Aug. 3, 1982; U.S. Pat. No. 4,463,045 entitled "Macroscopically Expanded Three-Dimensional Plastic Web Exhibiting Non-Glossy Visible Surface and Cloth-Like Tactile Impression", issued to Ahr, et al. on Jul. 31, 1984; and U.S. Pat. No. 5,006,394 "Multilayer Polymeric Film" issued to Baird on Apr. 9, 1991.

The absorbent core can be any absorbent member which is generally compressible, conformable, non-irritating to the wearer's skin, and capable of absorbing and retaining body fluids. The absorbent core may be manufactured in a wide variety of sizes and shapes (e.g., rectangular, hourglass, "T" -shaped, asymmetric, etc.) and from a wide variety of liquid-absorbent materials commonly used in disposable pull-on garments and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials.

The configuration and construction of the absorbent core may vary (e.g., the absorbent core may have varying caliper zones, a hydrophilic gradient, a superabsorbent gradient, or lower average density and lower average basis weight acquisition zones; or may include one or more layers or structures). Further, the size and absorbent capacity of the absorbent core may also be varied to accommodate wearers ranging from infants through adults. However, the total absorbent capacity of the absorbent core should be compatible with the design loading and the intended use of the absorbent article.

The absorbent core may include other optional components. One such optional component is the core wrap, i.e., a material, typically but not always a nonwoven material, which either partially or totally surrounds the core. Suitable core wrap materials include, but are not limited to, cellulose, hydrophilically modified nonwoven materials, perforated films and combinations thereof.

The backsheet can comprise a liquid impervious film. The backsheet can be impervious to liquids (e.g., body fluids) and can be typically manufactured from a thin plastic film. However, typically the backsheet can permit vapors to escape from the disposable article. In an embodiment, a microporous polyethylene film can be used for the backsheet. A suitable microporous polyethylene film is manufactured by Mitsui Toatsu Chemicals, Inc., Nagoya, Japan and marketed in the trade as PG-P.

One suitable material for the backsheet can be a liquid impervious thermoplastic film having a thickness of from about 0.012 mm (0.50 mil) to about 0.051 mm (2.0 mils), for example including polyethylene or polypropylene. Typically, the backsheet can have a basis weight of from about 5 g/m² to about 35 g/m². However, it should be noted that other flexible liquid impervious materials may be used as the backsheet. Herein, "flexible" refers to materials which are compliant and which will readily conform to the general shape and contours of the wearer's body.

The backsheet can be typically positioned adjacent the outer-facing surface of the absorbent core and can be joined thereto by any suitable attachment means known in the art. For example, the backsheet may be secured to the absorbent core by a uniform continuous layer of adhesive, a patterned layer of adhesive, or an array of separate lines, spirals, or spots of adhesive. Illustrative, but nonlimiting adhesives, include adhesives manufactured by H. B. Fuller Company of St. Paul, Minn., U.S.A., and marketed as HL-1358J. An example of a suitable attachment means including an open pattern network of filaments of adhesive is disclosed in U.S. Pat. No. 4,573,986 entitled "Disposable Waste-Containment Garment", which issued to Minetola et al. on Mar. 4, 1986. Another suitable attachment means including several lines of adhesive filaments swirled into a spiral pattern is illustrated by the apparatus and methods shown in U.S. Pat. No. 3,911,173 issued to Sprague, Jr. on Oct. 7, 1975; U.S. Pat. No. 4,785,996 issued to Ziecker, et al. on Nov. 22, 1978; and U.S. Pat. No. 4,842,666 issued to Werenicz on Jun. 27, 1989. Alternatively, the attachment means may include heat bonds, pressure bonds, ultrasonic bonds, dynamic mechanical bonds, or any other suitable attachment means or combinations of these attachment means as are known in the art.

The backsheet may be additionally secured to the topsheet by any of the above-cited attachment means.

The absorbent article may also include such other features (not shown) as are known in the art including, but not limited to, re-closable fastening system, lotion, acquisition layers, distribution layers, wetness indicators, sensors, elasticized waist bands and other similar additional elastic elements and the like, belts and the like, waist cap features, containment and aesthetic characteristics and combinations thereof.

In some embodiments, the absorbent article is a sanitary napkin or a pantiliner.

In one embodiment, the absorbent core of the absorbent article can comprise the layered structure comprising the foam layer and the reinforcing layer. In another embodiment, the absorbent core can further comprise a layer of superabsorbent polymer material which is positioned above the layered structure.

In one embodiment, the layered structure can be comprised between the topsheet and the absorbent core and can act as an acquisition layer or a distribution layer.

In a further alternative embodiment of the present invention, as illustrated in Figure 1, a layered structure comprising a melamine foam associated to a reinforcing layer can be incorporated in an absorbent article for feminine hygiene, for example a sanitary napkin or pantiliner, comprising a composite layered absorbent core similar to those described in our copending patent application EP 1447067, or patent application with application number EP 09154506.1, both applications assigned to The Procter & Gamble Company. The absorbent article can comprise a fluid pervious topsheet (30), for example a perforated film topsheet, a fluid impervious backsheet (40), for example a polyethylene film, optionally a secondary topsheet layer comprised between the topsheet and the backsheet, for example nonwoven made of bicomponent fibres, and a composite layered absorbent core (28) comprised between the secondary topsheet, when present, and the backsheet. The composite, layered core structure (28) comprises, from the topmost layer facing towards the topsheet, to the bottom layer facing towards the backsheet, a cover layer (130), for example constituted by a polypropylene spunbonded nonwoven with a basis weight of 28 g/m², a layer of fiberized hot melt adhesive (120), i.e. a layer of hot melt adhesive provided in form of microfibres, with a basis weight of 8 g/m², a layer of superabsorbent polymer material in particle form (110), with a basis weight of 144 g/m², and a substrate layer (100) typically constituted by a layered structure according to the present invention, constituted by a layered structure sold by the company Celso, France, under the trade name of soClean™. The layered structure in turn comprises, again from top to bottom: a hydraulically bonded nonwoven layer (140) with 70% viscose fibres and 30% polyester fibres, having a basis weight of 70 g/m² and a thickness of 710 µm, constituting the reinforcing layer, a fusible copolyamide nonwoven layer having a basis weight of 12 g/m², and a layer of melamine foam (150) having a basis weight of 8 g/m² and a thickness of 800 µm. The intermediate fusible copolyamide nonwoven layer is heat activated in order to bonds together the reinforcing layer (140) and the foam layer (150), which are therefore associated to each other.

The absorbent article comprising the layered structure according to the present invention incorporated in the absorbent core structure is soft and conformable, hence providing comfort in use, and at the same time is resilient and mechanically resistant. The core therefore, and in turn the whole article, has a better shape stability and integrity, also under the stresses experienced in use, and also upon absorption of fluid.

According to further alternative embodiments of the present invention, similar to the one described above, the layered structure comprising the foam layer and the reinforcing layer associated thereto can alternatively constitute the topmost cover layer of the composite layered core structure.

### Test Methods

### Thickness of the layered structure and of the respective layers

Methods for the measurement of the thickness of a layer of foam material or of a reinforcing layer, as well as of a whole layered structure comprising both of them, are well known in the art.

The thickness of a layer of a compliant material, such as a foam or a reinforcing layer according to the present invention, or a layered structure comprising them, can be suitably measured with methods which do not involve subjecting the sample material to any pressure, besides of course atmospheric pressure, and can typically be selected by the skilled person among optical methods, e.g. based on image analysis. Methods using Calibrated Image Analysis, or a Stage Micrometer are known and right at hand of the skilled person. The thickness of the selected layer may hence be accordingly measured on a cross section of the layer, for example on 25 mm x 25 mm square specimens cut from a layer of material, either from a standard sample of the material or from a sample taken from a finished product. Length and width of the specimens shall also be accurately measured for example with a digital calliper.

Optical methods, such as those mentioned above, can easily allow the skilled person to measure the thickness of the single layers as such, also when present within a composite material comprising the foam layer combined with a reinforcing layer, e.g. a fibrous reinforcing layer, as well as the overall thickness of a whole layered structure, for example typically in case a layered structure is taken from a commercial product. In order to prepare the specimens the cut shall be clean and perpendicular to the plane of the specimen, taking care that the edge be not pinched together or distorted by the cut. The cut shall be done with a sharp razor blade, for example typically cut in guillotine fashion to get a neat edge. At least two thickness measurements shall be made on each side of the specimen, being equally spaced along the side; the thickness of each specimen is obtained as the average of all thickness measurements. Specimens shall be cut from at least three products or samples, and their respective thicknesses averaged. The resulting value shall be taken as the thickness of the foam layer.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An absorbent article for feminine hygiene comprising a layered structure, **characterized in that** the layered structure comprises a melamine foam layer and a reinforcing layer associated to said melamine foam layer.

2. The absorbent article for feminine hygiene according to claim 1, wherein said melamine foam layer has a thickness of 0.2 to 1.2 mm, preferably between 0.5 to 1.0 mm.

3. The absorbent article for feminine hygiene according to any of the preceding claims, wherein said reinforcing layer has a thickness of 0.2 to 4.5 mm, preferably between 0.5 to 1.0 mm.

4. The absorbent article for feminine hygiene according to any of the preceding claims, wherein the overall thickness of said layered structure is of 0.5 to 5.0 mm, preferably between 0.85 to 2.0 mm.

5. The absorbent article for feminine hygiene according to any of the preceding claims, wherein said melamine foam layer is formed by cutting a melamine foam block by peeling.

6. The absorbent article for feminine hygiene according to any of the preceding claims comprising a topsheet, a backsheet and an absorbent core therebetween, wherein the absorbent core comprises said layered structure.

7. The absorbent article for feminine hygiene according to claim 6, wherein said absorbent core comprises a layer of superabsorbent polymer material positioned above said layered structure.

8. The absorbent article for feminine hygiene according to claims 1 to 5 comprising a topsheet, a backsheet and an absorbent core therebetween, wherein said layered structure is comprised between the topsheet and the absorbent core and acts as an acquisition layer.

9. The absorbent article for feminine hygiene according to any of the preceding claims, wherein said reinforcing layer of said layered structure is made of a nonwoven material.

10. The absorbent article for feminine hygiene according to any of the preceding claims, wherein the absorbent article is selected from the group consisting of sanitary napkins and pantiliners.

11. The absorbent article for feminine hygiene according to any of the preceding claims, wherein the absorbent article is a sanitary napkin.

12. The use of a layered structure comprising at least a melamine foam layer and a reinforcing layer associated to said melamine foam layer in an absorbent article for feminine hygiene.
